# EUROPEAN PATENT APPLICATION

(11) **EP 1 656 838 A1**
(43) Date of publication of application: **17.05.2006**
(21) Application number: 04772095.8
(22) Date of filing: 18.08.2004
(51) Int. Cl.: A23L 1/29, A61P 13/08, A61K 49/00

(54) **FOOD FOR TESTING LIFE STYLE-RELATED DISEASES**

(30) Priority: 21.08.2003 JP 2003297851
(71) Applicant: Abilit Corporation, Osaka-shi, Osaka 542-0081 (JP); Harano, Yutaka, Minoh-shi, Osaka 562-0042 (JP)
(72) Inventor: HARANO, Yutaka, 5620042 (JP)
(74) Representative: W.P. Thompson & Co.
(86) International application number: PCT/JP2004/012134
(87) International publication number: WO 2005/018341

(57) **Abstract**

It is intended to provide a food for testing life style-related diseases which is in the form of a food easy to chew, differing from the existing medicines such as Trelan G, and makes it possible to simultaneously and easily perform a glucose tolerance test and examine oral glucose tolerance, hyperlipemia after meal and so on. Namely, a test food for testing metabolic factors causing life style-related diseases which contains 100 parts by weight of carbohydrate and from 20 to 40 parts by weight of fat. It contains from 73 to 77 g of carbohydrate and from 15 to 30 g of fat, in terms of a single test dose, and provides from 460 to 600 kcal. This test food is usable in testing many items for diabetes, obesity, circulatory diseases, hyperlipemia, hyperuricemia, hyperinsulinemia and hypertension.

## Description

### Technical Field

The present invention relates to a food for testing a life style-related disease, and more particularly, to a food for testing which can be safely ingested orally and which can diagnose diabetes and glucose intolerance, and which can simultaneously and easily detect metabolic risk factors such as hyperinsulinemia, insulin resistance, hyperlipemia, etc.

### Background Art

In recent years, life style-related diseases have rapidly increased along with changes in life style such as eating habits and exercise. For example, diabetic patients including glucose intolerance (so-called prediabets) continue to increase, and when left as is, severe complications, such as angina pectoris, myocardial infarction, brain infarction, diabetic gangrene, diabetic neuropathy and retinopathy, renal failure, etc., occur. Since patients are unaware of their subjective symptom regarding their life style- related diseases because of gradual advancement, early detection of diseases is important.

Conventionally, testing for diabetes has been carried out by a glucose tolerance test. This test is a testing method in which a test solution containing 75 grams of glucose (which is designated as "Trelan G (trade name), "meaning Tolerance Glucose) is administered and blood sugar and insulin are measured by a time course. The course of treatment is decided on the basis of glucose tolerance which is judged from a pattern of change in blood sugar and insulin in blood obtained before a dose of Trelan G and 30 minutes, 60 minutes, 90 minutes, and 120 minutes after a dose of Trelan G.

Although Trelan G is a test solution approved by the Ministry of Health, Labor and Welfare of Japan as a medicament and used as a standard for glucose tolerance testing, too many factors, such as (1) the test solution is a decomposition product of starch andnotanaturalfood, (2) sometimes side effects, such as vomiting, diarrhea, sensation of abdominal distension, or disagreeable sensations are produced after ingestion, and (3) blood must be collected many times, are forced on examinees. In addition, data obtainable with Trelan G is limited to blood sugar and insulin, and only diabetes and glucose intolerance [IGT (impaired glucose tolerance), prediabets] can be diagnosed.

### Disclosure of the Invention

Glucose intolerance including diabetes, hyperlipemia, obesity, hypertension and insulin resistance as the common foundation have been attracting attention as main metabolic factors in life style-related diseases, and they are important also from the aspect of pathologic analysis. However, the glucose tolerance test using Trelan G has problems as described above. Therefore, the purpose of the invention is to provide a food for testing for simultaneously and easily detecting the above risk factors.

In order to attain the above purpose, the present inventors have completed a food for testing for early and simultaneous detection of multi-item metabolic factors in life style-related diseases as targeted by the invention, by advancing development of a food for testing which is in the form of a food easy to chew, differing from a medicament, which makes it possible to perform a glucose tolerance test, examine oral glucose tolerance, etc. Namely, the invention provides a food for testing for metabolic factors in life style-related diseases as described below.
(1) A food for testing for detecting metabolic factors in life style-related diseases containing 100 parts by weight of carbohydrates and from 20 to 40 parts by weight of fat.
(2) The food for testing for detecting metabolic factors in life style-related diseases according to the above described (1) containing from 73 to 77 grams of carbohydrates and from 15 to 30 grams of fat in terms of a single test ingestion.
(3) The food for testing for detecting metabolic factors in life style-related diseases according to the above described (2), which is in the form of a cookie and which provides from 460 to 600 kilocalories of ingestion calories in terms of a single test.
(4) The food for testing for detecting metabolic factors in life style-related diseases according to any one of the above described (1) to (3), in which a life style-related disease is one, two or more diseases selected from a group consisting of diabetes, obesity, circulatory disease, hyperlipemia, hyperuricemia, hyperinsulinemia, and hypertension.
(5) The food for testing for detecting metabolic factors in life style-related diseases according to any one of the above described (1) to (4), in which the metabolic factor (s) in a life style-related disease is one, two or more factors selected from a group consisting of glucose, insulin, apolipoprotein B, high density lipoprotein, low density lipoprotein, neutral fat and remnant-like lipoprotein, and insulin resistance.
(6) A diagnostic method for life style-related diseases which comprises subjecting an examinee to ingestion of food for testing for detecting metabolic factors in life style-related diseases containing 100 parts by weight of carbohydrates and from 20 to 40 parts by weight of fat, collecting blood from the examinee over a time course and testing for metabolic factors in life style-related diseases, and comparing results with a result obtained before ingestion, thereby learning of any change in metabolic factors in life style-related diseases and judging the presence or absence of an abnormal value.
(7) The diagnostic method for life style-related diseases according to the above described (6), in which a food for testing for detecting metabolic factors in life style-related diseases containing from 73 to 77 grams of carbohydrates and from 15 to 30 grams of fat in terms of a single test ingestion is administered.
(8) The diagnostic method for life style-related diseases according to the above described (7), in which a food for testing for detecting metabolic factors in life style-related diseases which is in the form of a cookie and which provides from 460 to 600 kilocalories of ingestion calories in terms of a single test is administered.

The food for testing for metabolic factors in life style-related diseases of the invention allows simultaneous and easy detection of main metabolic factors in life style-related diseases in place of Trelan G. In addition, since it is in the form of a food, it can be ingested for testing without a feeling of resistance.

### Best Mode for Carrying Out the Invention

The food for testing for metabolic factors in life style-related diseases of the invention (hereinafter, sometimes referred simply to as "food for testing") is in the form of a food and contains 100 parts by weight of carbohydrates and from 20 to 40 parts by weight of fat. The form of operations and processing such as baking or boiling of edible materials containing carbohydrates such as starch (and additionally containing partly maltose, etc.) and materials containing fat. The ratio of carbohydrates and fat should finally be the above-described ratio in the food for testing. The starch becomes digestible by changing into alpha-starch during the cooking operations and processing.

The edible materials containing carbohydrates such as starch and materials containing fat may be selected according to the purpose depending on the desired form of the food. For example, when the processing is directed at a cookie form, the main edible material containing carbohydrates (starch) is flour and the main material containing fat is butter. The edible form is not particularly limited and the cookie is preferred because of simple cooking operations and processing as well as ease in forming a predetermined shape and testing. For processing into a food, use of food materials other than materials containing carbohydrates and fat is permissible according to the desired food when needed for processing, and for example, a protein may be contained. In addition, partial content of natural metabolite such as maltose or glucose is permissible in the carbohydrates.

The food for testing of the invention contains carbohydrates and fat in the above-described ratio. When testing, an examinee receives the food so that from 73 to 77 grams (usually, the mean standard is 75 grams) of carbohydrates and from 15 to 30 grams of fat are ingested in terms of a single test ingestion. By adjusting the ingestion calories in terms of a single test to a value from 460 to 600 kilocalories, namely to ingestion calories of a normal breakfast, the examinee can ingest the food for testing as though he/she had eaten breakfast.

The method for testing metabolic factors in life style-related diseases with the food for testing of the invention may be carried out similar to the process with Trelan G. Namely, the presence or absence of an abnormal value is judged by checking for a change in a factor after collecting blood from the examinee before and after the ingestion of food for testing by a time course.

The life style-related diseases as possible targets in the invention are one, two or more diseases selected from the group consisting of diabetes, obesity, cardiovascular disease, hyperlipemia, hyperuricemia, hyperinsulinemia and hypertension. Use of the food for testing of the invention enables simultaneous and multi-item testing of metabolic factors relating to these diseases.

The metabolic factors for the above life style-related diseases include one, two or more factors selected from the group consisting of glucose, insulin, apolipoprotein B, high density lipoprotein, low density lipoprotein, triglyceride and remnant-like particles. Presence or absence is judged by comparing the test values with, for example, values in Table 1. For insulin, IRI (immunoreactive insulin, blood insulin), as well as AUCI (area under the curve of insulin) and AUCI × AUCG (product of area under the curve of insulin and area under the curve of glucose) as an indicator for insulin resistance are used as described in Table 1 as mentioned later. In Table 1, Apo-B denotes apolipoprotein B, HDL-C high density lipoprotein, LDL-Clow densitylipoprotein,HOMA Homeostasis Modelassessment, TGneutral fat, andRLP-Cremnant-likelipoprotein, respectively.

In the invention, indicators for various test results of metabolic factors in life style-related diseases are exemplified in Table 1.

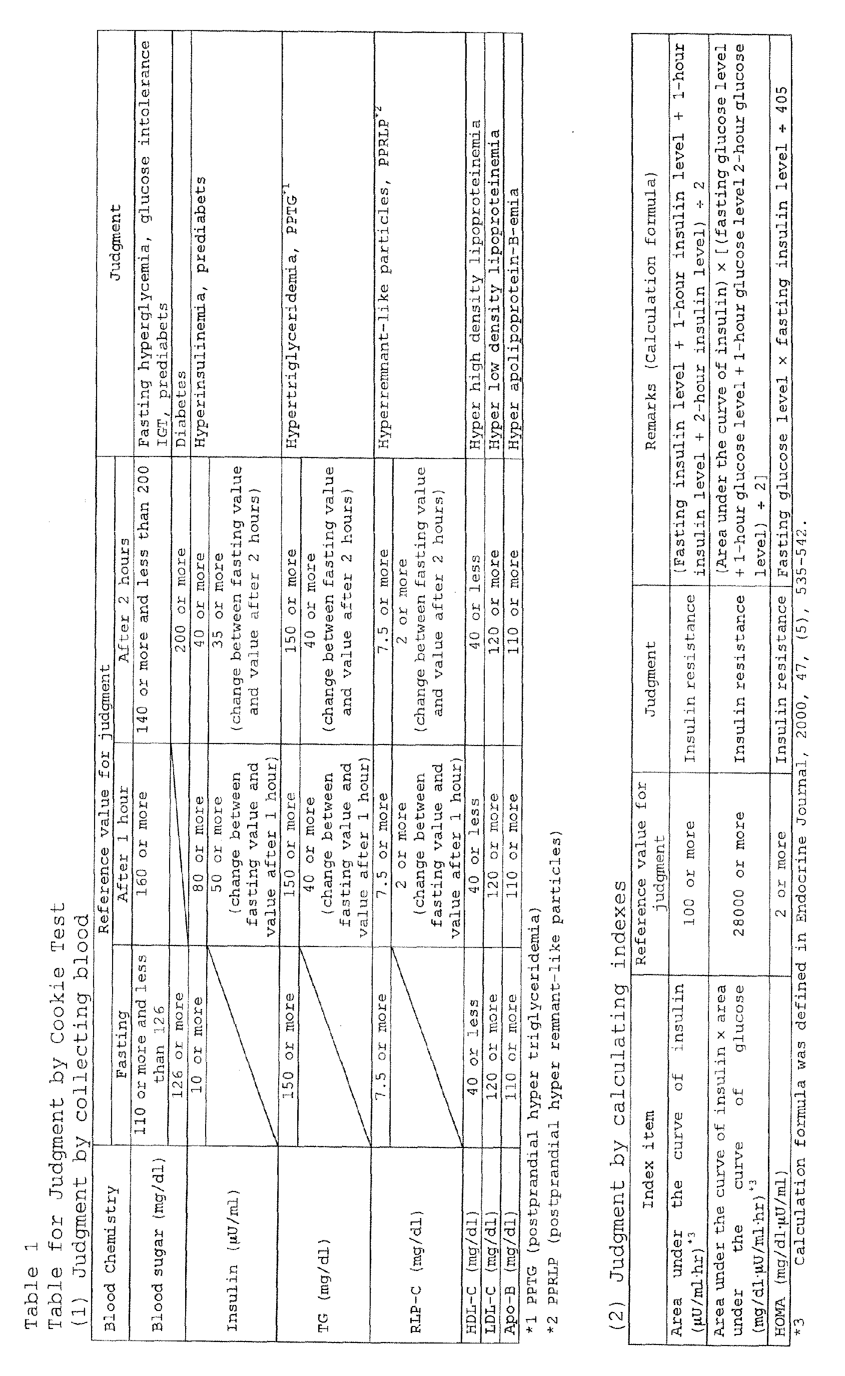

### EXAMPLES

The following Example and Test Examples will describe the invention more specifically.

### Example 1

According to the usual production method for cookies, crude materials containing 100g of weak flour, 30g of maltose, 45g of butter, 10g of whole egg, 5g of water, 0.5g of sodium bicarbonate and 0. 6g of ammonium carbonate per single ingestion were mixed, and baked at 180°C for 25 minutes to produce a cookie-like food.

This food was produced with weak flour as a starch source, butter as a fat ingredient, and a small amount of maltose for forming the desired shape, with the addition of egg. The cookie contains 74.3 g of carbohydrates, 25.2g of lipids and 7.0g of protein per single ingestion, and has a caloric energy of 551 kcal. The food is suitable for judgment of blood sugar, hyperlipemia particularly a high level after a meal, excess or low level of insulin secretion, ability for metabolic treatment of living organisms for sugar or fat after a meal, and efficacy of insulin concerning life style-related diseases.

### Test Example 1

A cookie containing 75g of wheat starch (containing 15% of maltose), 24g of fat and 7g of protein, and having a caloric energy of 560 kcal, was administered to a healthy group, acorpulent group, a group having an exercise regimen (at least 1 hour a day and 3 days or more a week), a group having a life style-related disease (containing subject with suspected life style-related disease). Blood sugar, IRI, TG, TC (total cholesterol), RLP-C, and Apo-B were measured by PAGE (polyacrylamide gel electrophoresis), lipoprotein analysis and leptin was measured. Breath analysis was carried out additionally. Hereinafter, this test is referred to as cookie test.

As a result, responses of hyper-insulinemia, hyper-triglyceridemia, and hyper-reminant-like-lipoproteinemia (postprandial dyslipidemia) were observed in juvenile obesity, indicating insulin resistance. For postprandial dyslipidemia, an increase in VLDL (very low density lipoprotein) was mainly present and a small dense formation of remnant and LDL was partly observed. A decrease in glucose oxidation and an increase in fatty acid oxidation were also observed. In the group having an exercise regimen, energy consumption at rest was low in significance, which is caused by a decrease in fatty acid oxidation. On the other hand, VO₂max (maximum oxygen uptake) clearly indicated a high value. Blood sugar and IRI responses in the cookie test showed low values, and the area under the curve of insulin, and product of area under the curve of insulin and area under the curve of glucose, as indicators showing insulin resistance advocated by Harano, et al. were low, indicating a remarkably high insulin sensitivity. Also, TG and RLP-C were lower in comparison with pre-ingestion values, and no postprandial dyslipidemia was observed.

Reference values for blood glucose and insulin responses, TG, RLP-C, LDL-C, HDL-C, Apo-B and diagnosis of hyperlipemia as well as an indicator for insulin resistance are shown in Table 1. Among cases where values of LDL-C and Apo-B exceeded the fasting reference after load, the case was judged an anomaly when HDL-C decreased. In electrophoresis with Lipophor, an increase in remnant and VLDL as well as small dense formation of LDL were also observed.

When the cookie test was performed in 40 cases with IGT, life style-related diseases and suspected cases, 5% in diabetes, 30% in glucose intolerance, 50% inpostprandial hyperinsulinemia, 18% inpostprandial dyslipidemia, 33 to 43% in frequencyof insulin resistance were observed as abnormalities other than abnormalities found in typical testing and no case was judged as normal.

From the results of the cookie test, the following findings were obtained:
(a) Aphysiological test reflecting daily life as compared with the conventional method can be performed.
(b) Although a feeling of fullness is frequent, gastrointestinal complaints are uncommon.
(c) A reference value for hyper-insulinemia can be established and the ability of secretion and activity of insulin in the presence of not only sugar but also a fatty acid can be evaluated.
(d) Standards for postprandial dyslipidemia have been established (TG, RLP-C, small dense LDL, and remnant).
(e) The activity of insulin on lipid metabolism is made more sensitive as compared with that on sugar metabolism. A simple insulin sensitivity test can be formed by evaluating both.
(f) The test is useful for detection of multi-item metabolic factors in life style-related diseases and indicators for countermeasures.
(g) When the test is used together with breath analysis, it is found that energy consumption at rest is low and effective, and particularly, the fatty acid oxidation is decreased in a group having an exercise regimen.
(h) In the group having the insulin resistance such as obesity, it is found that glucose oxidation is decreased and fatty acid oxidation is suppressed and decreased after load.
(i) In addition, for measurement of blood leptin, the value is low in the group having an exercise regimen, and it is suggested that the decrease participates in a decrease in energy consumption and promotion of appetite.

As a conclusion, the following can be mentioned:
(1) The testingmethod utilizing the cookie of the invention allows judgment of diabetes and glucose intolerance with the same diagnostic standard as that utilizing Trelan G (except pancreatic exocrine secretion disorder), and is useful as oral glucose tolerance test.
(2) Establishment of reference values for hyper-insulinemia during fasting, after 1 hour and after 2 hours is possible.
(3) Establishment of indicator for insulin resistance is possible (AUCI, AUCI×AUCG).
(4) Establishment of indicator for postprandial dyslipidemia is possible (increase of TG, RLP-C).

At the same time, the method enables detection and evaluation of multi-item risk factors, and is useful in early detection and as indicators for countermeasures against metabolic factors in life style-related diseases regarding diabetes reserve, obesity, circulatory disease, hyperlipemia, hyperuricemia, etc.

### Test Example 2

In order to elucidate the metabolic properties and mechanism in a long term exercise regimen by the cookie for testing of the invention and breath analysis, the following test was carried out.

Materials and method: Male students having or not having an exercise regimen (at least 1 hour and 3 days or more a week) were charged with running, and AT (anaerobic threshold) and VO₂max were measured. Energy consumption at rest was also measured. The test cookie of the invention (containing 75 g of carbohydrates mainly consisting of wheat starch and 24 goffat) was administered, and blood examination for glucose intolerance, postprandial dyslipidemia, hyper-insulinemia and insulin resistance as well as breath analysis were carried out.

Results: Energy consumption at rest in subjects having an exercise regimen (exercise group) was 800 to 1,100 kcal/day/m², which was lower by 20 to 40% than that in subjects having no exercise regimen (the non-exercise group). This was caused by a decrease in fatty acid oxidation. However, after ingesting the cookie, exogenous fat was well oxidized. AT was observed at 8.4 Mets in the exercise group and 6. 5 Mets in the non-exercise group. Oxygen uptake at VO₂max was 3,389 and 2,462 ml/min, respectively. Blood sugar responses during fasting and after ingestion of the cookie and also insulin responses were significantly lower in the exercise group, indicating that insulin sensitivitywas improved. Area under the curve of insulin, and product of area under the curve of insulin and area under the curve of glucose, as a measure of insulin resistance, were significantly lower, indicating that insulin sensitivity was further strengthened. TG during fasting was low in the exercise group. According to PAGE analysis, postprandial dyslipidemia, observed in the non-exercise group, was not observed in the exercise group. Serum level of leptin was significantly lower in the exercise group. It is considered that this is an explanation of low energy consumption and promotion of appetite.

Conclusion: The exercise group clearly showed a decrease in energy consumption at rest, which may contribute to lower fatty acid oxidation. Oxygen uptake inAT and VO₂max was increased more than the value of the non-exercise group, indicating that theyhad further effective energy use and higher physical ability. After ingestion of the cookie of the invention, exogenous fat was easily oxidized and an increase in mitochondrial activity was shown. Simultaneous decrease in insulin response and decrease in blood sugar responses indicate enhancement of insulin sensitivity. For TG during fasting and its increase after ingestion of the cookie, the increase in VLDL in the exercise group was improved via activated LPL, by improvement of the insulin sensitivity concerning lipid metabolism. The cookie for testing of the invention allows judgment of glucose intolerance with the same criterion as that for liquid glucose. In addition, postprandial dyslipidemia, hyperinsulinemia and insulin resistance can be evaluated. Furthermore, the invention can be widely used not only in testing of suspected diabetes but also in testing of obesity, hypertension, hyperlipemia and insulin resistance, cardiovascular diseases as well as general testing of metabolic risk factors in life style-related diseases.

### Industrial Applicability

The food for testing for metabolic factors in life style-related diseases of the invention is useful for detection of multi-item metabolic factors and as an indicator for judgment of the effect of countermeasures.

## Claims

1. A food for testing for detecting metabolic factors in life style-related diseases comprising 100 parts by weight of carbohydrates and from 20 to 40 parts by weight of fat.

2. The food for testing for detecting metabolic factors in life style-related diseases according to claim 1 comprising from 73 to 77 grams of carbohydrates and from 15 to 30 grams of fat in terms of single test ingestion.

3. The food for testing for detecting metabolic factors in life style-related diseases according to claim 2, wherein the food for testing for detecting metabolic factors in life style-related diseases is in the form of a cookie and provides from 460 to 600 kilocalories of ingestion calories in terms of a single test.

4. The food for testing for detecting metabolic factors in life style-related diseases according to any one of claim 1 to claim 3, wherein the life style-related disease is one, two or more diseases selected from the group consisting of diabetes, obesity, circulatory disease, hyperlipemia, hyperuricemia, hyperinsulinemia, and hypertension.

5. The food for testing for detecting metabolic factors in life style-related diseases according to any one of claim 1 to claim 4, wherein the metabolic factors in a life style-related disease is one, two or more factors selected from a group consisting of glucose, insulin, apolipoprotein B, high density lipoprotein, low density lipoprotein, triglyceride and remnant-like particles, and insulin resistance.

6. A diagnostic method for life style-related diseases, which comprises subjecting an examinee to ingestion of a food for testing for detectingmetabolic factors in life style-related diseases containing 100 parts by weight of carbohydrates and from 20 to 40 parts by weight of fat, collecting blood from the examinee by a time course and testing for metabolic factors in life style-related diseases, and comparing results with a result obtained before ingestion, thereby learning of any change in the metabolic factors in life style-related diseases and judging the presence or absence of the abnormal value.

7. The diagnostic method for life style-related diseases according to claim 6, wherein a food for testing for detecting metabolic factors in life style-related diseases containing from 73 to 77 grams of carbohydrates and from 15 to 30 grams of fat in terms of a single test ingestion is ingested.

8. The diagnostic method for life style-related diseases according to claim 7, wherein a food for testing for detecting metabolic factors in life style-related diseases is in the form of a cookie and provides from 460 to 600 kilocalories of ingestion calories in terms of a single test is ingested.
